# EUROPEAN PATENT APPLICATION

(11) **EP 3 936 080 A1**
(43) Date of publication of application: **12.01.2022**
(21) Application number: 20185065.8
(22) Date of filing: 09.07.2020
(51) Int. Cl.: A61B 90/20, A61B 34/20, A61B 90/00, A61B 17/00, G02B 21/00

(54) **NAVIGATED MEDICAL IMAGING**

(71) Applicant: Koninklijke Philips N.V., 5656 AG Eindhoven (NL)
(72) Inventor: HENDRIKS, Bernardus Hendrikus Wilhelmus, 5656 AE Eindhoven (NL); BABIC, Drazenko, 5656 AE Eindhoven (NL); SPLIETHOFF, Jarich Willem, 5656 AE Eindhoven (NL); HOLTHUIZEN, Ronaldus Frederik Johannes, 5656 AE Eindhoven (NL); HOMAN, Robert Johannes Frederik, 5656 AE Eindhoven (NL); VUURBERG, Edward, 5656 AE Eindhoven (NL); DRIES, Johan Juliana, 5656 AE Eindhoven (NL)
(74) Representative: Philips Intellectual Property & Standards

(57) **Abstract**

The present invention relates to providing information of a subject during interventional procedures. In order to improve the surgeon's vision during interventions, a navigated medical imaging system (10) is provided that comprises a medical imaging arrangement (12), a navigation arrangement (14), a microscope device (16) and a controller (20). The medical imaging arrangement is configured to provide image data representing interior structures of a region of interest of a subject (22). The navigation arrangement is configured to provide relative spatial tracking of the subject, the medical imaging arrangement and the microscope device. The microscope device is configured to provide at least one image of an exterior region of interest of the subject. The microscope device is mounted to the navigation arrangement. The controller is configured to determine a target view of the microscope device and to adjust the microscope device respectively to provide images according to the target view.

## Description

### FIELD OF THE INVENTION

The present invention relates to providing information of a subject during interventional procedures, and relates in particular to a navigated medical imaging system and to a method for providing image information of a subject.

### BACKGROUND OF THE INVENTION

During execution of surgical procedures, it may be important to maintain an optimal view of the surgical field that may significantly vary in size and depth, depending upon the type of executed therapy. Given the rise of minimally invasive surgery and ever-continuous tendency to provide for smaller incisions, it is expected that surgeries in the future will primarily be based on image-guided navigated systems where surgical wounds will be minimized down to a bare minimum for insertion of surgical instrumentation only. There are surgical procedures that require utilization of surgical microscope, where surgical resection of the brain tissue is reduced to a needed minimum - to provide for enlarged view through a craniotomy hole. Such surgical microscope systems may have a significant footprint in the operating room and may have relatively difficult maneuvering capability, high purchasing cost, and (almost) continuous need to adjust the microscope to either another angle of view or compensate for tissue motion. As an example, WO 2018/059837 A1 describes microscope tracking based on video analysis. Nevertheless, there are also surgical procedures that do not require utilization of a surgical microscope; here the surgical field is exposed to surgeon's vision. However, optimal viewing is required due to limited size of the surgical wound as well due to the incisional depth.

### SUMMARY OF THE INVENTION

There may thus be a need to improve the surgeon's vision during interventions.

The object of the present invention is solved by the subject-matter of the independent claims; further embodiments are incorporated in the dependent claims. It should be noted that the following described aspects of the invention apply also for the navigated medical imaging system and for the method for providing image information of a subject.

According to the present invention, a navigated medical imaging system is provided. The system comprises a medical imaging arrangement, a navigation arrangement, a microscope device and a controller. The medical imaging arrangement is configured to provide image data representing interior structures of a region of interest of a subject. The navigation arrangement is configured to provide relative spatial tracking of the subject, the medical imaging arrangement and the microscope device. The microscope device is configured to provide at least one image of an exterior region of interest of the subject. The microscope device is mounted to the navigation arrangement. The controller is configured to determine a target view of the microscope device and to adjust the microscope device respectively to provide images according to the target view.

The target view can also be referred to as desired view. The terms "target" or "desired" relate to parameters for a view that are predefined, or pre-set, by the user.

The provision of the microscope mounted to the navigation arrangement provides the effect that only one navigation system is needed and that a stable tracking system is provided. Instead of separate tracking of a separate microscope, the relative arrangement is easier to determine due to the mount or coupling of the microscope device to the navigation arrangement.

The microscope device thus provides the option to zoom in with the microscope, but to keep a distance such that enough space is provided in the actual field of interest, i.e. where the interventional device and the like are present and are handled during the intervention. In other words, a working distance is provided. The microscope device can thus be provided small and light. Due to the mount to the navigation arrangement, also the footprint of the microscope device is minimized.

According to an example, the navigation arrangement is configured to provide i) data representative of the spatial relation of the microscope device to the navigation arrangement. Further, the navigation arrangement is configured to provide at least one of the group of: ii) data representative of the spatial relation of the subject to the navigation arrangement, and iii) data representative of the spatial relation of the medical imaging arrangement to the navigation arrangement.

According to an example, the microscope device is rigidly fixed to the navigation arrangement, while the navigation arrangement is motorized such that the microscope device including the navigation arrangement is arranged to be movable.

The advantage is that the position of the microscope relative to the navigation is known to a higher precision than when the microscope can be moved with the navigation system.

According to another, alternatively, example, the microscope device is movably mounted to the navigation arrangement with a motorized mount such that the microscope device is arranged to be movable in relation to the navigation arrangement.

The advantage is that the position of the microscope relative to the navigation is known due to the control of the motorized movement.

Due to the resulting movability (indirectly and directly) of the microscope (for both examples), different views can be achieved.

According to an example, the microscope device is also part of the tracking cameras of the navigation arrangement.

In other words, apart from performing high magnification images of the surgical field, the microscope device contributes to tracking of the navigation arrangement. As an example, the microscope device is used to determine subject motion or brain shift motion; or the microscope device is used in device tracking. The high magnification can be used to increase the accuracy of the tracking.

According to the present invention, also a method for providing image information of a subject is provided. The method comprises the following steps:
- providing image data representing interior structures of a region of interest of a subject with a medical imaging arrangement;
- providing relative spatial tracking of the subject, the medical imaging arrangement and the microscope device with a navigation arrangement;
- providing at least one image of an exterior region of interest of the subject with a microscope device mounted to the navigation arrangement; and
- determining a target view of the microscope device and adjusting the microscope device respectively to provide images according to the target view.

According to an aspect, a medical microscope device is attached to a medical navigation arrangement. As an option, an X-ray system or MRI, CT, Spect-CT or ultrasound imaging system is used as well. As an example for an application area, the system is used in the surgery field where a surgical microscope is used, such as in neurosurgery.

According to an aspect, due to the coupling and co-registration of the surgical microscope with the pre-acquired imaging data sets, the microscope may be automatically moved to particular clinical details of interest, e.g. as seen in the images.

As an advantage for those surgical procedures that do not require utilization of a surgical microscope, an optimal viewing angle can be interactively taken, thus providing optimal wound visualization and sharpening the view in accordance with the acquired depth.

As an effect of the navigation arrangement and the microscope being provided as one system, a separate setup and alignment with respect to each other is not necessary.

An advantage of the smoothly integrated microscope device in combination with the navigation arrangement is that this allows viewing of relevant small structures during surgery.

As an option, relevant structures seen on images taken by the imaging system, such as by X-ray, MRI, CT or ultrasound or any other imaging system capable of imaging the inside of the body, are automatically found by the integrated microscope device.

According to an aspect, it is proposed to integrate a microscope device into a navigation arrangement. The microscope device comprises a camera with high magnification zoom lens placed on a motorized stage that allows orientation of the camera in four directions (x and y Cartesian direction and θ and ϕ angular directions). The camera position and angle are controlled by the navigation arrangement. Since the camera is attached to the navigation arrangement, the relation between the coordinate system of the camera and the navigation is known. Once the navigation arrangement is activated, the position of the subject is also known. Further, the relevant structures detected by the imaging system are now known by the navigation arrangement when the imaging coordinate system and the navigation coordinate system are coupled. The surgeon can now determine a relevant structure in the image of the imaging system and the microscope device will be pointed by adjusting the θ and ϕ angular directions of the motorized stage to this location controlled by the navigation arrangement. Preferably, the position x and y are selected by an algorithm linked to the navigation arrangement such that optimal view of the structure is possible.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a navigated medical imaging system.
Fig. 2 shows an example of a microscope device mounted to a navigation arrangement.
Fig. 3 shows an example of an interventional scenario with an example of the navigated medical imaging system in the context of a subject support and with a C-arm X-ray imaging system as an example for a medical imaging arrangement.
Fig. 4 shows basic steps of an example of a method for providing image information of a subject.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

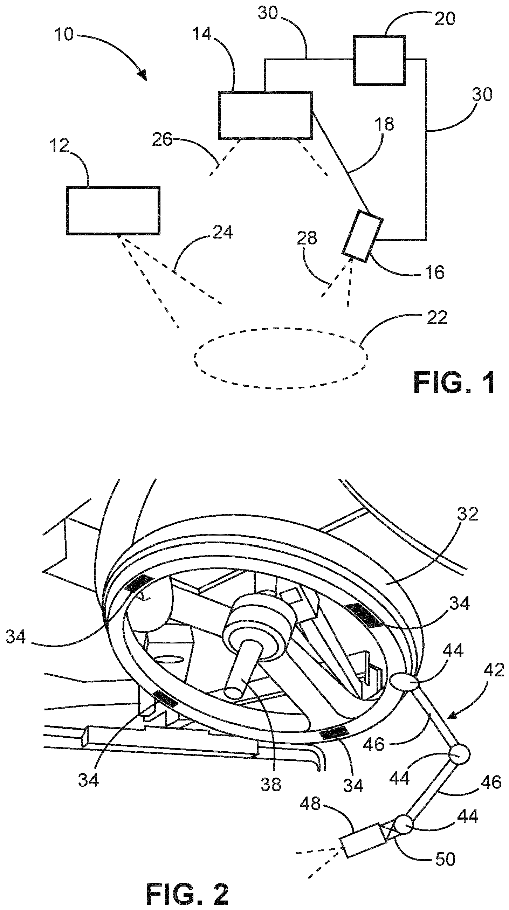

Fig. 1 schematically shows an example of navigated medical imaging system 10. The system 10 comprises a medical imaging arrangement 12, a navigation arrangement 14, a microscope device 16 and a controller 20. The medical imaging arrangement 12 is configured to provide image data representing interior structures of a region of interest of a subject 22. The navigation arrangement 14 is configured to provide relative spatial tracking of the subject 22, the medical imaging arrangement 12 and the microscope device 16. The microscope device 16 is configured to provide at least one image of an exterior region of interest of the subject 22. The microscope device 16 is mounted to the navigation arrangement 14. The mount is schematically indicated with connection mount line 18. The controller 20 is configured to determine a target view of the microscope device 16 and to adjust the microscope device 16 respectively to provide images according to the target view.

The image acquisition of the medical imaging arrangement 12 is schematically indicated with two hashed lines 24. The relative spatial tracking of the navigation arrangement 14 is schematically indicated with two further hashed lines 26. The provision of the at least one image of the exterior region of interest of the subject 22 of the microscope device 16 is schematically indicated with two other hashed lines 28. A data connection of the controller 20 to the navigation arrangement 14 and the microscope device 16 is indicated with data connection lines 30. The data connection can be provided wire-based or wireless.

The medical imaging arrangement 12 can also be referred to as medical imaging system, medical imaging device, imaging system or medical imager. The medical imaging arrangement 12 comprises an image acquisition device. The medical imaging arrangement can be provided as a mobile imaging system that is movable along the floor for example. The medical imaging arrangement can also be provided as a fixedly installed imaging system that is movably mounted to a floor, wall or ceiling structure.

The navigation arrangement 14 can also be referred to as navigation system, navigation device, positional tracking arrangement, spatial detection system or navigator.

The microscope device 16 can also be referred to as microscope system, microscope arrangement, magnifier, zoom camera or microscope. In an example, the microscope device 16 provides several images, e.g. a sequence of images, for example a stream of images like a live stream.

In an example, the microscope device 16 is provided as a stereo microscope. In an example, the microscope is a stereo microscope and the images are sent to a head mounted display such as the Microsoft Hololens.

The controller 20 can also be referred to as central link or processor that controls and coordinates the operation of e.g. the medical imaging arrangement 12, the navigation arrangement 14 and the microscope device 16.

In an example, the controller 20 (i.e. processor) receives data from the navigation, regarding the position of the subject and the relevant location to be visualized by the microscope device 16.

The microscope device 16 is configured to provide magnified images of the subject. In an example, the microscope device 16 comprises a camera with a high magnification zoom lens. As an example, a magnification factor of at least 10 is provided. As another example, a magnification factor of at least 100 is provided. As a further example, a magnification factor of at least 1000 is provided.

The target view, or desired view, relates to at least one of the group of position, viewing direction, angular direction, zoom factor and focus distance. The target / desired view relates to a view with pre-set or predetermined criteria, such as a certain viewing angle or range of angles, certain direction in relation to an interventional tool or the subject, such as minimal distortion, minimal concealed parts of the region of interest comprising the interventional tool and viewing direction aligned with at least one of the group of tool axis, viewing axis of the surgeon, normal to subject surface. The "desired view" refers to a predetermined view, e.g. set by the user beforehand or a view stored in a viewing setting parameter data storage. In an example, the desired view is a view in a predetermined relation to a viewing angle of the medical imaging arrangement. For example, the desired view is aligned with the view of the medical imaging arrangement, or perpendicular to the viewing axis of the medical imaging arrangement.

The term "target/desired view" can also relate to when there is a cavity in the body, for instance the burr hole in the skull, such that an optimal view inside the burr hole is provided. The term "target/desired view" can also relate to a view in which the hands of the physician are not obscuring the field of view. The term "target/desired view" can further also relate to a view towards a tumor to be resected or a certain structure (blood vessel, nerve etc.).

In an example, the microscope device 16 is capable of performing photoplethysmography imaging such that for instance local perfusion can be measured of the surgical area. Apart from this the microscope can also be provided with hyperspectral imaging and fluorescence imaging

In an example, the microscope device 16 provides a reduced field of view in size due to the higher resolution, but the missing information is replaced by navigation information provided by the navigation arrangement 14.

In an option, areas outside the field of view of the microscope device 16 are augmented with lower resolution. As an example, image data from the navigation arrangement 14, e.g. from one or more cameras, is used for an overview image.

The functionalities of a microscope device 16 are reduced, but the combination with the tracking system provides the missing functionalities.

In an option, the navigation arrangement 14 is configured to provide i) data representative of the spatial relation of the microscope device 16 to the navigation arrangement 14. The navigation arrangement 14 is further configured to provide at least one of the group of ii) data representative of the spatial relation of the subject 22 to the navigation arrangement 14, and iii) data representative of the spatial relation of the medical imaging 12 arrangement to the navigation arrangement 14.

The first data i) can be provided by sensors in case of a movable mount. In case of a fixed mount, i.e. rigid and not movable mount, the first data i) can be predetermined and provided to the system.

The second data ii) can be provided by sensors of the navigation arrangement 14 or by tracking of the subject 22.

The third data iii) can be provided by sensors of the navigation arrangement 14 or by tracking of the medical imaging arrangement 12.

In a first option, the microscope device 16 is rigidly fixed to the navigation arrangement 14 while the navigation arrangement 14 is motorized such that the microscope device 16 including the navigation arrangement 14 is arranged to be movable.

In an example, the microscope device 16 is mounted to the navigation arrangement 14 that has a motorized platform. The motorized platform is controlled by the controller 20 based on the relative spatial tracking provided by the navigation arrangement 14.

In a second option, the microscope device 16 is movably mounted to the navigation arrangement 14 with a motorized mount (not shown in detail in Fig. 2) such that the microscope device 16 is arranged to be movable in relation to the navigation arrangement 14.

Due to the resulting movability of the microscope device 16 (for both options), different views can be achieved. In examples, the microscope device 16 comprises at least one of the following cameras: camera for visual light, thermal camera, depth camera, hyperspectral camera and fluorescence imaging camera.

An example for the fluorescence imaging camera is 5-ALA fluorescent agent imaging used in neurosurgery, for example. Another example for the fluorescence imaging provides the use of Indocyanine green (ICG) fluorescent agent for surgical oncology. In an example, the navigation arrangement 14 is configured to track devices used during an intervention. The navigation arrangement 14 comprises at least one tracking camera for tracking the patient, i.e. the subject 22, and the medical imaging arrangement 12.

In an example, the navigation arrangement 14 comprises at least two cameras.

Fig. 2 shows an example of the microscope device 16 mounted to the navigation arrangement 14.

As an option, Fig. 2 shows an example of the navigation arrangement 14 that comprises a ring 32 with one camera 34, or two, three or four cameras 34, or more than four cameras. As an example, the tracking system 14 looks for markers and/or the subject. The ring 32 is mounted to a ceiling structure, for example by a movable mount 36 with one or more arms. A central handle 38 may be provided as an option to allow manual adjustment. Thus, it is possible to position the microscope device 16 to see the object in relevance to markers. The at least one tracking camera provides a tracking device.

In Fig. 2, the microscope device 16 is movably mounted to the navigation arrangement 14. As an example, a movable mount 42 comprises several joints 44 and arms 46 and a microscope 48 attached at the distal end thereof.

In an example, the microscope 48 is attached by a pan-tilt mechanism 50, which the user can control remotely (details not further shown) .

In an option, the microscope device 16 is also part of the tracking cameras of the navigation arrangement 14.

As an example, the navigation arrangement 14 comprises at least one camera, and the second required camera for tracking is provided by the microscope device 16. This provides the option of a loop-like control to correct the tracking. It is thus possible to calibrate the tracking system, i.e. the navigation arrangement 14, when a tracker is visible.

In an example, the microscope device 16 comprises a sensor, for instance one or more cameras, that determines the position of the microscope device 16 relative to the navigation arrangement 14.

In an example, the microscope device 16 has a guidance system such as a laser attached to the microscope that points at the subject. This point on the subject can be detected by the navigation arrangement 14 and be used in the control loop to orient the microscope in the correct position and orientation.

In an option, the navigation arrangement 14 is equipped with at least one 3D-depth camera that has a larger field of view such that a surrounding context can be provided to the user in addition to the image from the microscope device 16. In an example, the 3D-depth camera is a time-of-flight camera. In an example, the microscope image/s is/are combined with image data provided by the medical imaging system to generate overlay images or augmented reality. The overlay with pre-operational image date, like pre-op CT, for example, allows the determination of selection of the region of interest.

In an example, the microscope device 16 comprises an image stabilization system. Due to the large magnification, small vibration may cause image artefacts that could hamper the physician. The image stabilization prevents these image artefacts.

In a further option, the controller 20 is configured to detect subject motion based on the relative spatial tracking by the navigation arrangement 14 and to adapt the target view. The controller 20 is further configured to adjust the microscope device 16 when subject motion is detected. The controller 20 is also configured to detect at least one of breathing motion, blood pulsation and system vibrations, such that this information can be used to correct at least one of the microscope device's view and the visualization of the microscope device's video stream.

In another option, the controller 20 is configured to detect at least one of the group of instruments and other objects in the microscope device's field of view. The system 10 is able to automatically change the orientation of the microscope device 16 with respect to the region of interest such than an optimal view with minimal disturbance of other objects is achieved.

The optimal view relates to a view with pre-set or predetermined criteria, such as minimal distortion, minimal concealed parts of the region of interest comprising the interventional tool and viewing direction aligned with at least one of the group of tool axis, viewing axis of the surgeon, normal to subject surface.

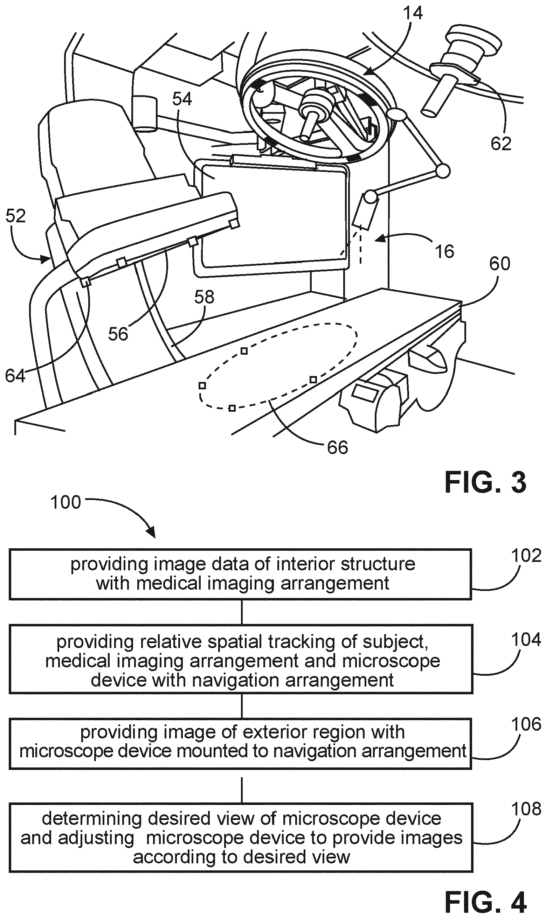

Fig. 3 shows an example of an interventional scenario with an example of the navigated medical imaging system 10 in the context of a subject support 40 and with a C-arm X-ray imaging system 52 as an example for the medical imaging arrangement 12. The C-arm can be a mobile system or a fixedly installed movable system.

As an option, shown in Fig. 3, but also provided for the examples of the system 10 in Fig. 1 and Fig. 2, a display 54 is provided configured to visualize at least the images provided by the microscope device 16. The display 54 may also be used for presenting other image data and information to the user.

In an option, the medical imaging arrangement 12 is provided as X-ray imaging system, such as the C-arm X-ray imaging system 52. The C-arm X-ray imaging system 52 is shown with a detector 56 mounted to one end of a C-arm 58. Further, a source is provided mounted to the other end of the C-arm, but not shown in Fig. 3.

In the foreground, as an option, a subject support 60 is indicated, for example supported by a motorized base to adjust height and inclination and horizontal positioning of a support surface for receiving a subject.

As an example, ceiling suspended lighting equipment 62 is also indicated.

In further options (not further shown), the medical imaging arrangement 12 is provided as an imaging arrangement of the group of CT imaging system, Spect-CT imaging system, magnetic resonance imaging system and ultrasound imaging system.

The mounting of the microscope device 16 to the navigation arrangement 14 provides exact knowledge of the microscope's position in relation to the navigation arrangement 14. For example, if the mount is provided as rigid mount, the spatial relation is also fixed and can thus be determined as a sort of calibration. For example, if the mount is provided as movable or adjustable, the spatial relation is variable but can easily be detected, e.g. constantly, by sensors arranged within the movable mount.

Hence, the microscope is within the navigation's spatial reference system, i.e. with the same reference space. The navigation then detects and determines the spatial relation to the medical imaging arrangement and to the subject. By this, all different parts of interest are within the same reference space and each of their spatial relation is known. This allows a facilitated adjustment of the microscope to be able to provide improved imaging in view of the user's needs, i.e. to improve the surgeon's vision during interventions.

In an example, the integrated microscope-navigation arrangement is provided in the context of a fixed hybrid room segment. As an example, the integrated microscope-navigation arrangement is provided for the Azurion system by Philips. Such system may be provided with integrated surgical navigation in the system detector and in the slave navigation device. Such system can also be used in combination with another imaging modality, such as a mobile X-ray C-arm system, MRI, CT, PET or ultrasound system.

In a first example, the navigation arrangement is the surgical navigation arrangement of Philips, for example with cameras 64 attached to the detector (as an option), where four cameras are provided attached to a movable base structure of the navigation arrangement. Optionally, markers 66 can be attached to the subject, e.g. a patient, for easy subject tracking. The microscope device is attached to the navigation system by a direction manipulating device (for adjusting the θ and ϕ angular directions) and position manipulating device (for x and y movement). In this system, the coordinate system of an X-ray system is directly coupled to the navigation arrangement. As a result, the positions of the relevant structures in the images taken by the X-ray system can now directly be used to guide the microscope device to that direction. Furthermore, the microscope image can be augmented with the 3D X-ray image.

In a second example, steering the microscope's view based on surgeon's head movement is provided. As a difference, the head movement of one or more surgeons is tracked and used to steer the microscope's viewing direction. The tracking of the head movement can be achieved by using the navigation arrangement to use markers attached to the surgeon's head or accessory on the surgeon's head (e.g. glasses, surgical cap). Another way would be to use image-processing methods to automatically track anatomical markers of the surgeon's head (e.g. eyes, ears), for example by using deep learning, machine learning, artificial intelligence etc. Based on the surgeon's head movement, the microscope's position, orientation and focus is adjusted such that tissue can be viewed from another angle or distance. In case of multiple surgeons, the surgeon(s) can assign one surgeon as "master" to steer the microscope. Preferably, each of the surgeons can take over control by using a simple method that does not require the surgeon to touch anything. For example, by making a hand gesture that is recognized by the navigation arrangement, giving a voice command, or making a certain head movement (e.g. a nod with the head).

In a third example, tracking and visualization using surgical headset is provided. As a difference, a headset (e.g. Microsoft Hololens) is used to adjust the microscope's position, orientation and focus; and to display the microscope video stream. The surgeon's head movement is tracked by using build-in inertia sensors of the display. The surgeon can steer the viewing direction relative to the tissue of interest by making slight head movements. For example, slight tilting / rotation of the head are used to look from a slightly different angle to the tissue. Optionally, the navigation arrangement is used to track the surgeon's head(set); this can then be used as additional feed for the microscope's adjustment system. For example, the surgeon can move his/her head slightly forward or backward to zoom in or out. As above, in case of multiple surgeons, the surgeon(s) can tell the system to use one or multiple headset as "master" to steer the microscope. Preferably, each of the surgeons can take over control by using a simple method that does not require the surgeon to touch anything. For example, by making a hand gesture that is recognized by the navigation arrangement, giving a voice command, or making a certain head movement (e.g. a nod with the head).

In another example, the microscope device has a motorized camera mount system. The mount can comprise a pan-tilt mechanisms. By a position sensor on every motorized axis, the position of the microscope camera is known accurately and can also be controlled accurately by means of feedback position control.

An example of how to use the system defined above is to take an image of the interior of the body with the X-ray system; to load X-ray image in navigation arrangement and translate the image into the coordinate system of the navigation arrangement (this translation is known due to the fact that the navigation arrangement and the X-ray system are physically connected); to start tracking subject; to determine relevant locations for microscopic view in the X-ray image and to translate these into the coordinate system of the navigation arrangement; to determine, based on X-ray image and location of the subject (which is determined by navigation arrangement), where to aim the microscope device; and to aim the microscope device to location the subject and to correct aiming when the subject moves according to the navigation arrangement.

Fig. 4 shows basic steps of an example of a method 100 for providing image information of a subject. In a first step 102, also referred to as step a), image data is provided representing interior structures of a region of interest of a subject with a medical imaging arrangement. In a second step 104, also referred to as step b), relative spatial tracking of the subject, the medical imaging arrangement and the microscope device is provided with a navigation arrangement. In a third step 106, also referred to as step c), at least one image of an exterior region of interest of the subject is provided with a microscope device mounted to the navigation arrangement. In a fourth step 108, also referred to as step d), a target view of the microscope device is determined and the microscope device is adjusted respectively to provide images according to the target view.

The first, second and third step can be provided simultaneously or in a different order. The fourth step can be provided simultaneously or subsequently.

The term "subject" may also be referred to as individual. The "subject" may further also be referred to as patient, although it is noted that this term does not indicate whether any illness or disease is actually present with the subject.

In an example, not further shown, a computer program is provided enabling a processor to carry out the method of the examples above.

In an example, a computer program or program element for controlling an apparatus according to one of the examples above is provided, which program or program element, when being executed by a processing unit, is adapted to perform the method steps of one of the method examples above.

In another example, also not further shown, a computer readable medium having stored the program element of the example above.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A navigated medical imaging system (10), comprising:
- a medical imaging arrangement (12);
- a navigation arrangement (14);
- a microscope device (16); and
- a controller (20);
wherein the medical imaging arrangement is configured to provide image data representing interior structures of a region of interest of a subject (22);
wherein the navigation arrangement is configured to provide relative spatial tracking of the subject, the medical imaging arrangement and the microscope device;
wherein the microscope device is configured to provide at least one image of an exterior region of interest of the subject;
wherein the microscope device is mounted to the navigation arrangement; and
wherein the controller is configured to determine a target view of the microscope device and to adjust the microscope device respectively to provide images according to the target view.

2. System according to claim 1, wherein the navigation arrangement is configured to provide:
i) data representative of the spatial relation of the microscope device to the navigation arrangement;
and at least one of the group of:
ii) data representative of the spatial relation of the subject to the navigation arrangement; and
iii) data representative of the spatial relation of the medical imaging arrangement to the navigation arrangement.

3. System according to claim 1 or 2, wherein the microscope device is rigidly fixed to the navigation arrangement while the navigation arrangement is motorized such that the microscope device including the navigation arrangement is arranged to be movable.

4. System according to claim 1 or 2, wherein the microscope device is movably mounted to the navigation arrangement with a motorized mount such that the microscope device is arranged to be movable in relation to the navigation arrangement.

5. System according to one of the preceding claims, wherein the microscope device comprises at least one of the following cameras:
- camera for visual light;
- thermal camera;
- depth camera;
- hyperspectral camera; and
- fluorescence imaging camera.

6. System according to one of the preceding claims, wherein the navigation arrangement is configured to track devices used during an intervention; and
wherein the navigation arrangement comprises at least one tracking camera for tracking the subject and the medical imaging arrangement.

7. System according to one of the preceding claims, wherein the microscope device is also part of the tracking cameras of the navigation arrangement.

8. System according to one of the preceding claims, wherein the navigation arrangement is equipped with at least one 3D-depth camera that has a larger field of view such that a surrounding context can be provided to the user in addition to the image from the microscope device.

9. System according to one of the preceding claims, wherein the controller is configured to detect subject motion based on the relative spatial tracking by the navigation arrangement and to adapt the target view;
wherein the controller is configured to adjust the microscope device when subject motion is detected; and
wherein the controller is configured to detect at least one of breathing motion, blood pulsation and system vibrations, such that this information can be used to correct at least one of the microscope device's view and the visualization of the microscope device's video stream.

10. System according to one of the preceding claims, wherein the controller is configured to detect at least one of the group of instruments and other objects in the microscope's field of view;
wherein the system is able to automatically change the microscope orientation with respect to the region of interest such than an optimal view with minimal disturbance of other objects is achieved.

11. System according to one of the preceding claims, wherein a display (54) is provided configured to visualize at least the images provided by the microscope device.

12. System according to one of the preceding claims, wherein the medical imaging arrangement is provided as an imaging arrangement of the group of X-ray imaging system (52), CT imaging system, Spect-CT imaging system, magnetic resonance imaging system and ultrasound imaging system.

13. A method (100) for providing image information of a subject, the method comprising the following steps:
- providing (102) image data representing interior structures of a region of interest of a subject with a medical imaging arrangement;
- providing (104) relative spatial tracking of the subject, the medical imaging arrangement and the microscope device with a navigation arrangement;
- providing (106) at least one image of an exterior region of interest of the subject with a microscope device mounted to the navigation arrangement; and
- determining (108) a target view of the microscope device and adjusting the microscope device respectively to provide images according to the target view.

14. A computer program enabling a processor to carry out the method of claim 13.

15. A computer readable medium having stored the program element of claim 14.
